# EUROPEAN PATENT APPLICATION

(11) **EP 2 015 076 A1**
(43) Date of publication of application: **14.01.2009**
(21) Application number: 07112274.1
(22) Date of filing: 11.07.2007
(51) Int. Cl.: G01N 33/68

(54) **Protein labelling with tags comprising isotope-coded sub-tags and isobaric sub-tags**

(71) Applicant: Koninklijke Philips Electronics N.V., 5621 BA Eindhoven (NL)
(72) Inventor: The designation of the inventor has not yet been filed
(74) Representative: Plünnecke, Ingo

(57) **Abstract**

Subject of the present invention are new isotopic and isobaric tagged molecules, kits comprising them and methods of using them in mass spectrometry.

## Description

### FIELD OF THE INVENTION

The purpose of this invention disclosure is to provide a technical solution for the proteome-wide screening for biomarkers and molecular targets by mass spectrometric approaches.

The basic idea presented herein is to provide a method, which allows for the parallel screening of at least up to 8 individual samples by LC-MS (liquid chromatography coupled to mass spectrometry) to search for disease relevant biomolecules in a high number of samples.

This is achieved by the combination of established isotopic and isobaric peptide labelling procedures and an appropriate experimental design. The isotopic tagging of the investigated peptides allows for the fast screening of expression differences between two disease groups on the level of MS, whereas the isobaric tagging of peptides allows for the relative quantification of different individual members of the two investigated groups on the level of MS/MS. Further, the data-dependent selection of MS signals for MS/MS investigation significantly increases throughput, as the number of MS/MS investigations is focused to potentially disease-relevant bio-molecules.

In particular, subject of the present invention are new isotopical and isobaric tagged molecules, kits comprising them and methods of using them in mass spectrometry.

### BACKGROUND OF THE INVENTION

### Protein separation techniques

### 2D-GE (two-dimensional gel electrophoresis)

The analysis and identification of proteins from highly complex mixtures demand tremendous resolving power. There are two different approaches. These include two-dimensional gel electrophoresis (2D-GE), and (two-dimensional) liquid chromatography (2D-LC). 2D-GE is unsurpassed in resolution (>5000 proteins), but suffers from lack of automation and reproducibility. Furthermore, some proteins such as hydrophobic membrane proteins, basic and acidic proteins, and very large or small proteins are poorly resolved. Since many promising disease markers show such properties, 2D-LC provides a good alternative for the high-resolution separation of complex protein mixtures (Hanash, S. et al (2003), Nature, 422, 226-232; Lipton M.S. et al (2002), Proc. Natl. Acad. Sci. USA, 99, 11049-11054). High-resolution 2D-GE was introduced 1975 (Klose J. (1975), Humangenetik, 26, 231-243; O'Farrell P.H. (1975), J. Biol. Chem., 250. 4007-40021). The first dimension of the procedure (isoelectric focusing) is exquisitely sensitive to molecular charge and the second dimension (SDS electrophoresis) is sensitive to polypeptide length, 2D-GE is very effective at revealing genetic variants (about one-third of which differ in net charge from wild type), proteolytic cleavages, and variations in sialic acid content. It has been widely used during the last decade as a tool for proteomic and biomarker analysis (Wittke, S. et al (2004), J. Chromat. B., 803, 17-26).

### 2D-LC (two-dimensional liquid chromatography)

2D-LC approaches are more suitable than 2D-GE to separate smaller proteins and the possibility for automation as well as throughput are significantly better. Several technologies to separate protein/peptide digests by liquid chromatography have been described, including capillary electrophoresis (CE) (Kasicka, V. (1999), Electroph., 20, 3084-3105), reversed-phase (RP)-HPLC, and two-dimensional liquid chromatography. Usually, for two-dimensional LC separation of protease-digested samples, strong cation exchange (SCX, first dimension) and reversed-phase HPLC (second dimension) chromatography are combined (Nägele, E. et al (2003), J. Chromatogr. A., 1009, 197-205) to resolve samples of more than 1000 proteins (Ross P.L. et al (2004), Molec. Cell. Proteomics, 3(12), 1154-69).

The two-dimensional liquid chromatography technology provides flexibility for purification and analysis strategies (Washburn M.P. et al (2001), Nat. Biotechnol., 19(3), 242-47; Peng J. et al (2003), J. Proteome Res., 2, 43-50). The LC component of 2D-LC generally uses ion-exchange columns (usually, strong cation exchange, SCX) back-to-back with reversed phase capillaries, operated in a series of cycles. In each cycle the salt concentration is increased in the ion-exchange column, in order to elute peptides according to their isoelectric points into the reversed phase system. In the following part of the cycle the hydrophobic conditions in the RP-HPLC are increased, to achieve maximal peptide separation resolution.

Many parameters influence the resolution power and subsequently the number of proteins that can be displayed by LC-MS (Nägele, E. et al (2004), J. Biomol. T., 15, 134-143). Usually, the 'on-line' configuration between the first-dimension separation technique (SCX) and the second-dimension RP-HPLC separation approach is set up for sample fractionation. As this requires a stepwise elution-gradient in the first dimension with a limited resolving power, the 'off-line' configuration, wherein the separation in the first and second dimension is uncoupled, is the preferred option. This allows improved sample resolution in the first dimension by applying a continuous, linear elution-gradient. Micro-fractions are collected, enriched on a C₁₈ trapping column, before they are applied to a reversed phase HPLC column.

### Mass spectrometry (MS)

Mass measurements by spectrometry are performed by the ionisation of analytes into the gas phase. A mass spectrometric instrument consists of three components, an ion source in order to vaporise the molecules of interest, a mass analyser, which determines and measures the mass-to-charge ratio (m/z) of the ionised molecules, and a sensor that registers and counts the number of ions for each individual m/z value. Each feature in an MS spectrum is defined by two values, m/z and a measure on the number of ions, which reached the detector of the instrument.

Two common techniques exist to ionise proteins or peptides for mass analysis in a spectrometer: electro-spray ionisation (ESI) and matrix-assisted laser desorption/ionisation (MALDI) (Fenn J.B. et al (1989), Science 246, 64-71; Karas M & Hillenkamp F, (1988), Anal. Chem., 60, 2299-2301).

Whereas ESI ionises the analytes out of solution and is therefore often directly coupled to liquid- chromatographic separation tools (e.g., RP-HPLC), MALDI pulses dry samples mixed with small organic molecules like cinnamic acid vaporises via laser. MALDI-MS is normally used to analyse relatively simple peptide mixtures, whereas integrated liquid-chromatography MS systems (LC-MS) are preferred for the analysis of complex samples.

The mass analyser is a key component of the technology; important parameters are sensitivity, resolution, mass accuracy and the ability to generate information-rich ion mass spectra from peptide fragments (tandem mass or MS/MS spectra) (Pandey A. & Mann T. (2000), Nature, 405, 837-846; Aebersold R. & Goodlett D.R. (2001), Chem. Rev. 101, 269-295). There are four basic types of mass analyser currently used in proteomics. These include the ion trap, time-of-flight (TOF), quadrupole and Fourier transform ion cyclotron (FT-MS) analysers. They are very different in design and performance, each with its own strengths and weaknesses.

### Quantification of mass spectrometry (MS) signals

The most relevant problem in the discovery of statistically significant protein expression differences between two samples (e.g., disease vs. control/healthy) by mass spectrometry technologies is the relative quantification of MS signals derived from different individual samples. Usually, the experimental variability (or the experimental noise) is in the order of CV>25-30% (CV = Coefficient of Variance, a measure of the experimental error or variability). This requires a large number of samples to be investigated to come to statistically significant results.

Therefore, it is highly desirable to develop methodologies, which allow the parallel processing of a multitude of individual samples in parallel in order to reduce the technical variability between different individual samples as much as possible. Recently, two approaches have been introduced to achieve this:

### Isotope-Coded Affinity Tag (ICAT™)

The recently developed isotope-coded affinity tag (ICAT^{™}) (Gygi, S.P. et al (1999), Nat. Biotechnol., 17, 994-999) technology allows for quantitative proteomic analysis based on differential isotopic tagging of related protein mixtures. The ICAT™ procedure uses three functional elements: a thiol-reactive chemical for the selective labelling of reduced cysteine residues, a biotin affinity tag to allow for selective isolation of labelled peptides, and an isotopic tag, which is synthesised in two isotopic forms, either normal "light" or "heavy" form (utilizing D or ¹³C). Typically, protein disulfide bridges are reduced under denaturing conditions before a protein sample is protease digested. The free thiol groups of the proteins from the two related samples to be compared are labelled respectively with the isotopically "light" or "heavy" forms of the reagent. The samples are combined, and then proteolysed with trypsin and the cysteine-containing peptides can be isolated via affinity chromatography. As only a moderate number of generated peptides will contain cysteine in its primary sequence, the complexity of the digested sample will again be decreased dramatically.

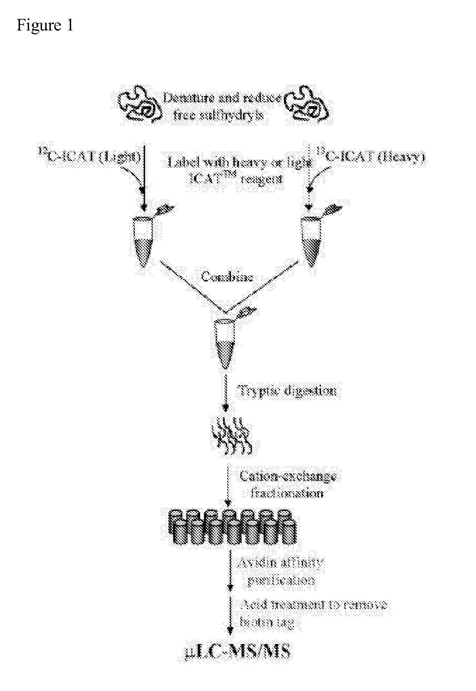

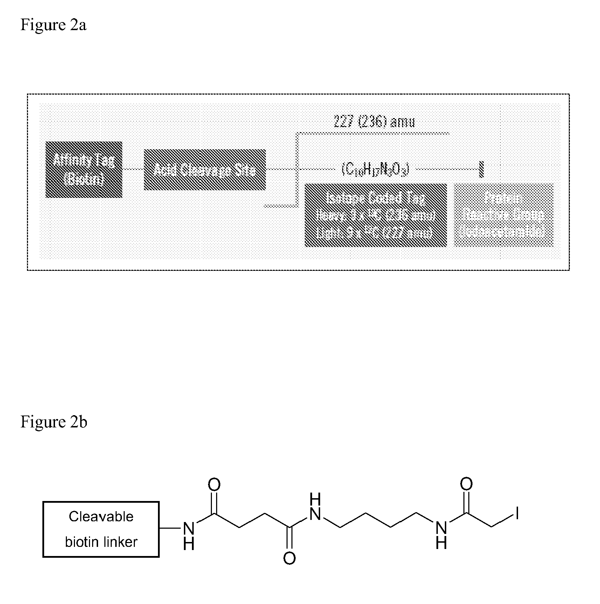

Two individually labelled peptide samples ("light" vs. "heavy") are combined before they are applied to separation techniques like liquid chromatography (Figure 1). This eliminates technical variability normally introduced by peptide separation techniques. The use of ¹²C/¹³C isotopic labels prevents a shift of retention time of peptides on RP-HPLC chromatography. Recently, an acid-cleavable isotope-coded affinity tag (cICAT) was introduced, which allows the straightforward affinity isolation of cysteine containing peptides (Figure 2a, b).

The identification of corresponding peptides in MS spectra is achieved by the high mass accuracy of high-resolution mass spectrometers that allow the exact matching of the 9 amu (atomic mass units) difference between the different isotopically tagged peptides with high precision.

### Isobaric Tags for Relative and Absolute Quantification (iTRAQ™)

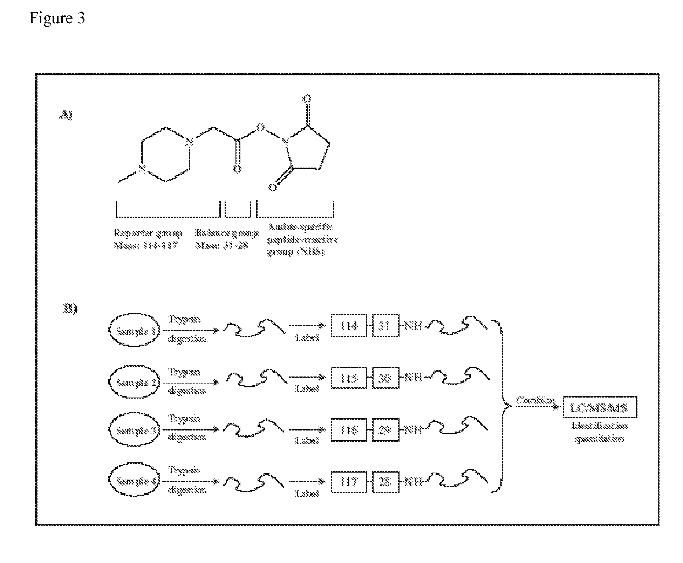

Recently, an amine group-based isotope labelling methodology, termed isobaric tags for relative and absolute quantification (iTRAQ^{™}), was developed (Ross, P.L. et al (2004), Mol. Cell. Proteomics, 3, 1154-1169). The iTRAQ^{™}reagent consists of a reporter group, a balance group and a peptide reactive group (Figure 3). The peptide-reactive group specifically reacts with primary amine groups of peptides similar to the method described above. The reporter group is a tag with a mass of 114, 115, 116 or 117 Da, depending on differential isotopic combinations of ¹²C/¹³C and ¹⁶O/¹⁸O in each individual reagent. The balance group ranges in mass from 28 to 31 Da to ensure that the combined mass of the reporter and balance groups remains constant (145 Da) for all four reagents. Therefore, peptides labelled with different isotopes are isobaric and are chromatogram-phically indistinguishable, a factor that is important for accurate quantification. During collision-induced dissociation (CID), the reporter group ions fragment from the backbone peptides, displaying distinct masses of 114 to 117 Da. The intensity of these fragments is used for quantification of the individual representative peptides. Unlike other stable isotope labelling approaches that use MS spectra for quantification, iIRAQ^{™} quantifies the relative peptide abundance from MS/MS spectra. The most significant advantage of this technology is that it allows labelling of up to four different samples within a single experiment. This four-multiplex labelling strategy is useful for quantifying proteins from multiplex samples, such as those in a time course study, replicate measurements of the same sample, or simultaneous comparison of normal, diseased and drug treated samples, in addition, the isotopically labelled peptides are isobaric and all contribute to one ion species that is observed in the MS and used for CID. This results in increased signal intensity and an increased probability of correct peptide identification, particularly for non-abundant proteins, which are often biologically meaningful.

Absolute and relative quantification of MS signals is an unsolved issue in biomarker discovery by technologies based on mass spectrometry approaches. However, the identification of relevant expression differences between, e.g., different disease groups like disease vs. control/healthy needs highly reproducible techniques, as relevant biomarkers can only be derived from a multitude of measurements.

The above described ICAT™ and iTRAQ^{™} techniques allow the multiplexing of either two (ICAT^{™}), or up to four (iTRAQ^{™}) individual samples and process them in parallel in order to minimise the technical variability. Nevertheless, these approaches have significant limitations. The ICAT™ approach only allows the investigation of two different samples, or two disease groups, which normally does not represent the full spectrum of disease relevant groups (e.g., different stages of progression of a disease). Therefore, it would be very advantageous to have a technique available, which allows the investigation of more than two groups. The iTRAQ^{™} technology is in principle ideally suited for this - however, the clear disadvantage of this technology is the need to perform MS/MS scans on each MS signal for relative quantification of the reporter signals. In practice, this is not feasible in case the starting material is of high complexity, like human serum, or other body fluids.

The present invention overcomes these limitations by a combination of the two individual techniques, by choosing an appropriate experimental design (see example below), and by applying a chemical peptide labelling technique, which allows introducing an isotopic and an isobaric tag into a peptide in a single step-procedure (see below for technical details).

### SUMMARY OF THE INVENTION

An essential feature of the invention is a protein/peptide labelling step with a 'tag', which combines the properties of isotopic, as well as isobaric tags. This combination allows for a simple one-step protein/peptide-labelling protocol, which otherwise is not possible to do.

Subject-matter of the present invention is a molecule for the detection, identification and/or quantification of one or more biomolecules in a sample, wherein said molecule is selected from a group comprising molecules of formula I, II, III, IV and V: 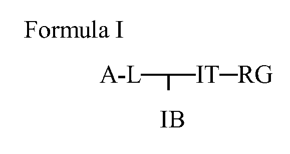 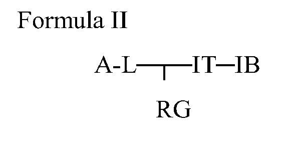 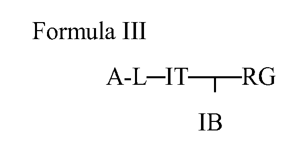 A-L-IB-IT-RG Formula IV

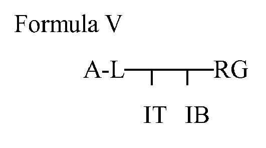

wherein A specifies at least one functional group for the reversible, covalent or non-covalent binding to a support material; IB is a group comprising an isobaric tag group; IT is a group comprising an isotopic tag group; L specifies a linker containing a cleavable moiety; and RG is a reactive group for the selective binding of biomolecules such as, but not limited to proteins or peptides. In a preferred embodiment of the invention the isobaric group is located at the terminal end of the inventive molecules as shown in formula I, II, III and V.

In a preferred embodiment of the present invention, IB comprises a predetermined breaking point between the isotopically labelled reporter group and an isotopically labelled counterbalancing group, whereby the molecule breaks upon induction by collision induced fragmentation.

A subject matter of the present invention is a kit comprising the molecules according to the invention.

A subject-matter of the present invention also is a kit comprising a combinatoric mixture of n · m ≥ 4-8 different molecules such as described for the formulas I, II, III, IV and V, whereby the integer n ≥ 2 specifies the number of differently labelled isotopic tag groups IT, each one having a different molecular weight, and the integer m ≥ 2-4 specifies the number of differently labelled isobaric tag groups IB, whereby all IB groups have the same molecular weight, while the counterbalancing group of each IB has a different molecular weight, and the combinations of the groups IT and IB are chosen such, that the highest possible number of permutations is achieved. The different mixtures of isotopes are chosen such that there is a characteristic molecular weight difference between individual isotopic tags. In that sense, it is relatively easy to identify corresponding peak pairs in an MS spectrum.

n · m ≥ 4-8 means that the product of n · m is definitely equals or is more than 4, preferred equals or is more than 6 and even more preferred equals or is more than 8, m > 2-4 means that m in any case equals or is more than 2, preferred equals or is more than 3 and even more preferred equals or is more than 4.

By using appropriate numbers of isotopes (several C, several N, several O) it is in principle possible to have more than only 2 isotopic components, e.g. 4 or 6. That would give rise to analyse much more samples in parallel; e.g. 4 / 6 isotopic X 4 isobaric =16 / 24. In a preferred embodiment of the kit n · m ≥ 8, n ≥ 2 and m ≥ 4.

Another subject-matter of the present invention is a kit which may comprise parts of a molecule such as described for the formulas I, II, III, IV and V, whereas one part of said molecules comprises the functional group IB, and another part comprises the functional group IT.

Subject matter of the invention is also a method for the detection, identification and/or quantification of one or more biomolecules in a sample.

Thus, another subject-matter of the present invention is a method for the detection, identification and/or quantification of one or more biomolecules in a sample, wherein said method comprises the steps of:
a) providing a sample which contains one or more biomolecules;
b) providing a combinatoric mixture of n · m ≥ 4-8 different molecules which are one or more derivatives of the molecule according to the invention; hereby the integer n ≥ 2 specifies the number of differently labelled isotopic tag groups IT, each one having a different molecular weight, and the integer m ≥ 2-4 specifies the number of differently labelled isobaric tag groups IB, whereby all IB groups have the same molecular weight, while the counterbalancing group of each IB has a different molecular weight, and the combinations of the groups IT and IB are chosen such, that the highest possible number of permutations is achieved; the different mixtures of isotopes are chosen such that there is a characteristic molecular weight difference between individual isotopic tags. In that sense, it is relatively easy to identify corresponding peak pairs in an MS spectrum;
c) labelling of the one or more biomolecules from step a) by attachment to the molecules of step b);
d) combining all samples from step c);
e) selecting the one or more biomolecules from the combined sample of step d), under the employment of functional group A of the molecules which are one or more derivatives of the molecule according to the invention, followed by removal of the one or more unbound biomolecules, and thereafter optionally releasing the bound biomolecules from the support material and the elution from the matrix via modification of the linker group by chemical or physical methods;
f) detecting, quantifying and/or identifying the one or more labelled biomolecules by means of LC-MS and/or LC-MS/MS.

In a preferred embodiment of the method n · m ≥ 8, n ≥ 2 and m ≥ 4.

### BRIEF DESCRIPTION OF THE DRAWINGS

- Figure 1: Proteomic flow-chart for quantitative analysis of protein profiles with cleavable ICAT™ reagent (Li, J. et al (2003), Molecular & Cellular Proteomics, 2, 1198-1204)
- Figure 2a: Schematic structure of cleavable ICAT™ reagent
- Figure 2b: chemical structure of cleavable ICAT™ reagent
- Figure 3: A) Chemical structure for reagents of isobaric tags for relative and absolute quantification, consisting of a reporter group with a mass ranging from 114 to 117 Da, a balance group with a mass ranging from 31 to 28 Da and an amine-specific peptide-reactive group. B) Strategy for quantification by iTRAQ^{™}. Protein mixtures from up to four different samples are proteolytically digested by trypsin, and resulting peptides are reacted with iTRAQ^{™}reagents (114-117), respectively. The labelled peptides are combined and analysed by liquid chromatography and mass spectrometry (LC-MS/MS) for quantification and identification.
- Figure 4: General outline of the workflow to investigate 2 groups, each consisting of 4 samples. 2 different isotopic tag groups IT and 4 isobaric tag groups IB are combined to enable the individual labelling of a total of 8 samples (see text for more details).

### DETAILED DESCRIPTION OF EMBODIMENTS

An essential feature of the invention is a protein/peptide labelling step with a 'tag', which combines the properties of isotopic, as well as isobaric tags. This combination allows for a simple one-step protein/peptide-labelling protocol, which otherwise is not possible to do. Subject-matter of the present invention is a molecule for the detection, identification and/or quantification of one or more biomolecules in a sample, wherein said molecule is selected from a group comprising molecules of formula I, II, III, IV and V:

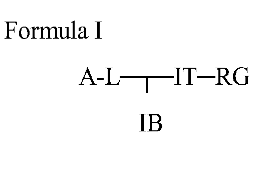

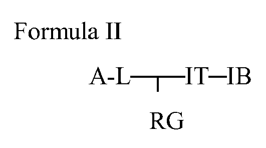

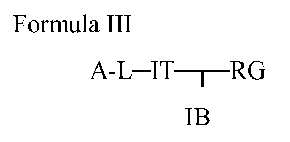

A-L-IB-IT-RG Formula IV

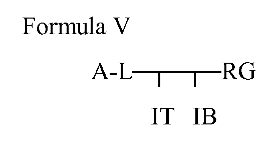

wherein A specifies at least one functional group for the reversible, covalent or non-covalent binding to a support material; IB is a group comprising an isobaric tag group; IT is a group comprising an isotopic tag group; L specifies a linker containing a cleavable moiety; and RG is a reactive group for the selective binding of biomolecules such as, but not limited to proteins or peptides. In a preferred embodiment of the invention the isobaric group is located at the terminal end of the inventive molecules as shown in formula I, II, III and V.

Support material hereby may encompass in one embodiment a substance comprising a chemical or a biomolecule, which is suitable for the affinity-driven selection of samples tethered to an according counterpart of the support material.

By this means substances labelled with a molecule according to the present invention can selectively be separated from unbound substances in a sample. Said support material may be, bound and/or immobilised on a solid surface, such as a column material, a functionalised resin, a glass surface or a plastic surface. A person skilled in the art may choose an appropriate combination of support material and its according counterpart. An example of this is the pair of avidin/biotin.

The support material can also be used as a mediator that itself is selectively bound to an appropriate secondary support. Again, a person skilled in the art can choose an appropriate secondary support. One example of this kind of secondary support is an antibody which is selective for the support material described above. This kind of interaction is for example utilised in the well-known ELISA method.

In a preferred embodiment of the present invention the solid support material is an immobilised biomolecule.
In a more preferred embodiment of the present invention the solid support material is immobilised avidin.
In a more preferred embodiment of the present invention the solid support material is immobilised streptavidin.
In a more preferred embodiment of the present invention the solid support material is immobilised neutravidin.
In another preferred embodiment of the present invention the solid support material is an immobilised biomolecule bound to a column material.
In another preferred embodiment of the present invention the solid support material is an immobilised biomolecule bound to a resin material.

In the claimed molecule of the present invention the functional group A may be selected from the group comprising biotin and derivatives thereof, hexa-histidine-tag, amylose binding protein, maltose binding protein, chitin, chitin binding protein, streptavidin, avidin, neutravidin, glutathione-S-transferase and green-fluorescent protein. The functional group is not limited to the before-mentioned embodiments.

In a preferred embodiment of the present invention the functional group A is biotin and derivatives thereof.

Functional group L of the molecule presented herein may be an aliphatic hydrocarbon chain that contains a cleavable moiety, the latter may be selected from the group comprising an acid labile moiety, a base labile moiety, a moiety which is cleavable by UV irradiation, a moiety which is cleavable by microwave irradiation, a moiety which is cleavable by change of electric potential, a moiety which is cleavable by oxidation, a moiety which is cleavable by reduction, a moiety which can be altered by olefine metathesis and a moiety which can be altered by disulfide exchange.

In a preferred embodiment of the present invention L contains a cleavable moiety which is selected from the group comprising disulfide moieties and acid labile moieties.
In a particularly preferred embodiment of the present invention L contains an acid labile moiety as a cleavable moiety.

In another particularly preferred embodiment of the present invention L contains a disulfide moiety as a cleavable moiety.

In the molecule which is subject-matter of the present invention the functional group IB comprises an isotopically labelled reporter group and an isotopically labelled counterbalancing group.

Hereby, IB can be labelled with various atoms which occur in more than one stable isotopic form.
The functional group IB may be labelled with the hydrogen isotopes ¹H and/or ²H.
The functional group IB may also be labelled with the carbon isotopes ¹²C and/or ¹³C.
The functional group IB may also be labelled with the nitrogen isotopes ¹⁴N and/or ¹⁵N.
The functional group IB may also be labelled with the oxygen isotopes ¹⁶O and/or ¹⁸O.
The functional group IB may comprise a heterocyclic moiety.

The functional group IB of the molecule may comprise an isotopically labelled reporter group and an isotopically labelled counterbalancing group as mentioned above.
More specifically, IB may comprise a predetermined breaking point between the isotopically labelled reporter group and the isotopically labelled counterbalancing group. In a preferred embodiment of the present invention, IB comprises a predetermined breaking point between the isotopically labelled reporter group and an isotopically labelled counterbalancing group, whereby the molecule breaks upon induction by collision induced fragmentation.

In a more preferred embodiment of the present invention the functional group IB is an N-Alkyl-N-Acetyl-1,4-Piperazine derivative.

In a particularly preferred embodiment the structure of the isobaric group IB is as shown in Figure 3 A) (Ross, P.L. et al (2004), Mol. Cell. Proteomics, 3, 1154-1169)
The functional group IT of the molecule presented herein can be an aliphatic hydrocarbon chain.

The functional group IT may be labelled with various atoms which occur in more than one stable isotopic form.

The functional group IT may be labelled with the hydrogen isotopes ¹H and/or ²H.
The functional group IT may also be labelled with the carbon isotopes ¹²C and/or ¹³C.
The functional group IT may also be labelled with the nitrogen isotopes ¹⁴N and/or ¹⁵N.
The functional group IT may also be labelled with the oxygen isotopes ¹⁶O and/or ¹⁸O.

In the molecule disclosed in the present invention, the functional group RG which is a reactive group for the selective binding of biomolecules such as, but not limited to proteins or peptides, can be selected from the group comprising amino-reactive chemical groups, thiol-reactive chemical groups, hydroxyl-reactive chemical groups and carboxyl-reactive chemical groups.

In a preferred embodiment RG is a thiol-reactive chemical group.
In a more preferred embodiment RG is iodoacetamide.
In another more preferred embodiment RG is a maleimide.
In another preferred embodiment RG amine-reactive chemical group.
In a more preferred embodiment RG is an NHS-ester.
In another more preferred embodiment RG is an imidoester.

It is important to note at this point, that some combinations of cleavable linker groups L and reactive groups RG will lead to problems due to inter- or intramolecular reactivity of the molecules disclosed in the present invention with themselves. An example for this would be the combination of a disulfide-containing linker L and a thiol-reactive group RG. A person skilled in the art will have to take this fact into account upon designing molecules as disclosed in the present invention, and avoid such unfavourable combinations. Therefore, L and RG are chosen in a way that avoids inter- or intramolecular reactivity of the molecules with themselves. The before said also applies to the other groups of the inventive molecules. A person skilled in the art would consider this without being explicitly told.

A subject matter of the present invention is a kit comprising the molecules according to the invention.

A subject-matter of the present invention also is a kit comprising a combinatoric mixture of n · m ≥ 4-8 different molecules such as described for the formulas I, II, III, IV and V, whereby the integer n ≥ 2 specifies the number of differently labelled isotopic tag groups IT, each one having a different molecular weight, and the integer m ≥ 2-4 specifies the number of differently labelled isobaric tag groups IB, whereby all IB groups have the same molecular weight, while the counterbalancing group of each IB has a different molecular weight, and the combinations of the groups IT and IB are chosen such, that the highest possible number of permutations is achieved. The different mixtures of isotopes are chosen such that there is a characteristic molecular weight difference between individual isotopic tags. In that sense, it is relatively easy to identify corresponding peak pairs in an MS spectrum.

n · m ≥ 4-8 means that the product of n · m is definitely equals or is more than 4, preferred equals or is more than 6 and even more preferred equals or is more than 8. m > 2-4 means that m in any case equals or is more than 2, preferred equals or is more than 3 and even more preferred equals or is more than 4.

By using appropriate numbers of isotopes (several C, several N, several O) it is in principle possible to have more than only 2 isotopic components, e.g. 4 or 6. That would give rise to analyse much more samples in parallel; e.g. 4 / 6 isotopic X 4 isobaric = 16 / 24. In a preferred embodiment of the kit n · m ≥ 8, n ≥ 2 and m ≥ 4.

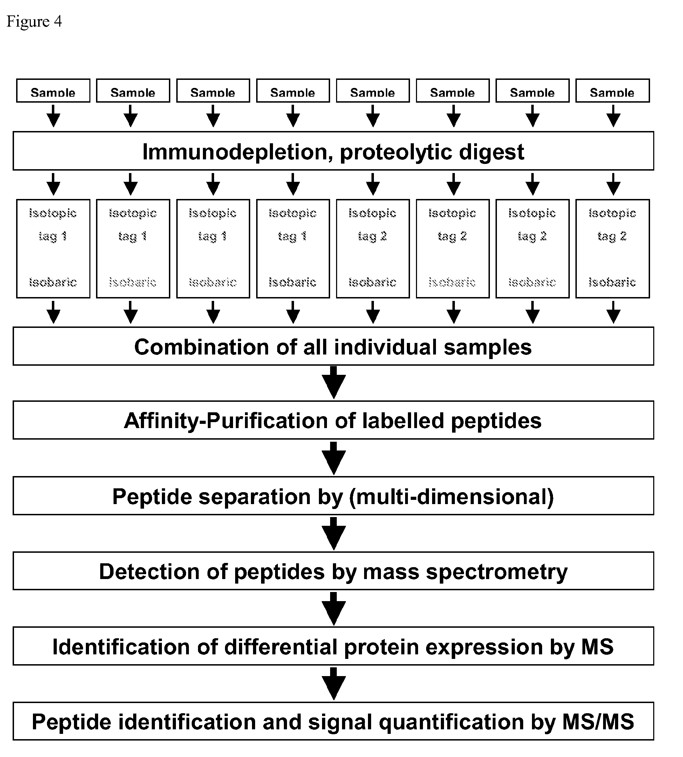

Figure 4 shows an example of the use of such a combinatoric mixture in the analysis of 2 groups, each consisting of 4 samples, by the method according to the present invention. Here, 2 different isotopic tag groups IT and 4 isobaric tag groups IB are combined in such manner that each IT group (IT 1 or IT 2 of Fig. 4) is combined with all possible IB groups (IB 1, IB 2, IB 3 or IB 4 of Fig. 4). This combination renders a total of 8 individual molecules according to the present invention, thus a total of 8 individual samples can be labelled in this example.

In a preferred embodiment of the kit as claimed by the present invention the integer n which specifies the number of differently labelled isotopic tag groups IT has a value of 2 and the integer m which specifies the number of differently labelled isobaric tag groups IB has a value of 4.

Another subject-matter of the present invention is a kit which may comprise parts of a molecule such as described for the formulas I, II, III, IV and V, whereas one part of said molecules comprises the functional group IB, and another part comprises the functional group IT.

The kit, as claimed by the present invention, may also comprise parts of a molecule such as described for the formulas I, II, III, IV and V, whereby one part comprises the functional groups IB, A, L and RG, and another part comprises the functional groups IT, A, L and RG.

Subject matter of the invention is also a method for the detection, identification and/or quantification of one or more biomolecules in a sample.

The general workflow for the investigation of 2 groups, where each group consists of 4 samples is outlined in Figure 4. The overall procedure comprises the following different steps:
1) In a first step, the protein extract of up to 8 individual samples is prepared for the down-stream processing and labelling with isotopic/isobaric tags, e.g., by immuno-affinity depletion of high-abundance proteins, protein denaturation, proteolytic digest, etc
2) In a next step, the samples will be divided into two relevant groups (e.g., disease vs. control/healthy), and the peptides (e.g., the cysteine-containing peptides) of the individual samples of each group (up to four) are tagged with a isotopic/isobaric coded affinity tag. For group 1 the isotopic tag 1 ("light") is used, whereas for group 2 the isotopic tag 2 is used ("heavy"). Groups 1 and 2 can therefore be discriminated in a single LC-MS run by a characteristic mass difference, occurring on the MS level. For the 4 individual samples within group 1 or group 2, the labelling compounds with the isobaric tag a, b, c, d are used respectively. In that sense, the 4 samples for each individual group can be discriminated after peptide fragmentation on the MS/MS level.
3) All samples are combined and the labelled peptides of the individual samples are purified via affinity column chromatography (e.g., avidin, streptavidin, Ni²⁺-chelated NTA (Nitrilotriacetic acid), etc).
4) Then, peptides are separated through multi-dimensional liquid chromatography, or other suitable techniques.
5) Finally, all fractions are investigated by mass spectrometry, and resulting spectra are investigated for differential peptide expression.
6) On identification of differentially expressed peptides, MS/MS scans will be performed for quantification of iTRAQ^{™} signals, and for peptide *de novo* sequencing.

Subject of the invention is also a more generalized method for the detection, identification and/or quantification of biomolecules in a sample.

Thus, another subject-matter of the present invention is a method for the detection, identification and/or quantification of one or more biomolecules in a sample, wherein said method comprises the steps of:
a) providing a sample which contains one or more biomolecules;
b) providing a combinatoric mixture of n · m ≥ 4-8 different molecules which are one or more derivatives of the molecule according to the invention; hereby the integer n ≥ 2 specifies the number of differently labelled isotopic tag groups IT, each one having a different molecular weight, and the integer m ≥ 2-4 specifies the number of differently labelled isobaric tag groups IB, whereby all IB groups have the same molecular weight, while the counterbalancing group of each IB has a different molecular weight, and the combinations of the groups IT and IB are chosen such, that the highest possible number of permutations is achieved; the different mixtures of isotopes are chosen such that there is a characteristic molecular weight difference between individual isotopic tags. In that sense, it is relatively easy to identify corresponding peak pairs in an MS spectrum;
c) labelling of the one or more biomolecules from step a) by attachment to the molecules of step b);
d) combining all samples from step c);
e) selecting the one or more biomolecules from the combined sample of step d), under the employment of functional group A of the molecules which are one or more derivatives of the molecule according to the invention, followed by removal of the one or more unbound biomolecules, and thereafter optionally releasing the bound biomolecules from the support material and the elution from the matrix via modification of the linker group by chemical or physical methods;
f) detecting, quantifying and/or identifying the one or more labelled biomolecules by means of LC-MS and/or LC-MS/MS.

In a preferred embodiment of the method n · m ≥ 8, n ≥ 2 and m ≥ 4.

In the first step of the mass spectrometric: analysis (MS), the ion of interest may be selected, fractionated and identified by a second step (MS/MS), including protein database searches in order to characterise the ion.

In a preferred embodiment of the method according to the present invention the integer n which specifies the number of differently labelled isotopic tag groups IT has a value of 2 and the integer m which specifies the number of differently labelled isobaric tag groups IB has a value of 4.

The preparation of samples covers the isolation of cultured cells, laser micro-dissected cells, body tissue, body fluids, or other relevant samples of interest. The preparation method to be used differs depending on the type of sample to be prepared for further investigation of its protein content, but standard procedures are usually available and known to the expert.

Cell lysis is the first step in cell fractionation and protein purification and as such opens the door to a myriad of biological studies. Many techniques are available for the disruption of cells, including physical and detergent-based methods. Historically, physical lysis has been the method of choice for cell disruption; however, it often requires expensive, cumbersome equipment and involves protocols that can be difficult to repeat due to variability in the apparatus (such as loose-fitting compared with tight-fitting homogenisation pestles). In recent years, detergent-based lysis has become very popular due to ease of use, low cost and efficient protocols.

All cells have a plasma membrane, a protein-lipid bilayer that forms a barrier separating cell contents from the extracellular environment. Lipids comprising the plasma membrane are amphipathic, having hydrophilic and hydrophobic moieties that associate spontaneously to form a closed bimolecular sheet. Membrane proteins are embedded in the lipid bilayer, held in place by one or more domains spanning the hydrophobic core. In addition, peripheral proteins bind the inner or outer surface of the bilayer through interactions with integral membrane proteins or with polar lipid head groups. The nature of the lipid and protein content varies with cell type.

Clearly, the technique chosen for the disruption of cells, whether physical or detergent-based, must take into consideration the origin of the cells or tissues being examined and the inherent ease or difficulty in disrupting their outer layer(s). In addition, the method must be compatible with the amount of material to be processed and the intended downstream applications.

Protein extraction may also include the pre-fractionation of cellular proteins originated from different compartments (e.g., membrane proteins vs. cytosolic proteins vs. proteins from other cell organelles like the nucleus, mitochondria, etc), or the pre-fractionation of proteins into sub-fractions according to their isoelectric point, molecular weight, etc.

In another preferred embodiment of the method according to the present invention the samples are biological samples.

In a more preferred embodiment of the method according to the present invention samples are biological samples derived from human blood, human body fluids, human tissue, human bone or human expulsions.

In another embodiment of the method according to the present invention step a) optionally comprises the immunodepletion of one or more biomolecules in said samples. In another embodiment of the method according to the present invention step a) comprises cleaving the one or more biomolecules in said sample.

In a preferred embodiment of the method according to the present invention a) comprises cleaving the one or more biomolecules in said sample with one or more proteolytic enzymes selected from the group of Asp-N Endopeptidase, Caspase 1, Caspase 2, Caspase 3, Caspase 4, Caspase 5, Caspase 6, Caspase 7, Caspase 8, Caspase 9, or Caspase 10, Chymotrypsin, Clostripain, Enterokinase, Factor Xa, Glutamyl Endopeptidase, Granzyme B, LysC Lysyl Endopeptidase, Pepsin, Proline-Endopeptidase, Proteinase K, Staphylococcal peptidase I, Thermolysin, Thrombin and Trypsin.

In a particularly preferred embodiment of the method according to the present invention wherein step a) comprises cleaving the one or more biomolecules in said sample by tryptic digest.

In another embodiment of the present invention the biomolecules of the sample, which are peptides and/or proteins are denatured before applying the proteolytic enzymes in step a).

In a more preferred embodiment of the present invention the biomolecules of the sample, which are peptides and/or proteins are denatured before applying the proteolytic enzymes in step a) by using a reagent from the group comprising guanidinium chloride and urea.

In a particularly preferred embodiment of the present invention the biomolecules of the sample, which are peptides and/or proteins are denatured before applying the proteolytic enzymes in step a) by using guanidinium chloride.

In a particularly preferred embodiment of the present invention the biomolecules of the sample, which are peptides and/or proteins are denatured before applying the proteolytic enzymes in step a) by using urea.

In one embodiment of the present invention the peptides resulting after applying the proteolytic enzymes in step a) are modified by guanidinylation or acetylation. This step is meant to cap free α- and ε-amino groups of the peptides.

In a preferred embodiment of the present invention the peptides resulting after applying the proteolytic enzymes in step a) are modified by guanidinylation.

In a preferred embodiment of the present invention the peptides resulting after applying the proteolytic enzymes in step a) are modified by acetylation.

In a more preferred embodiment of the method, which is subject-matter of the present invention, the biomolecules are selected from the group comprising a pyrogen, a pharmaceutical compound, a pharmaceutical lead compound, an allergen, an autoimmunogen, a toxin, a polyclonal antibody, a monoclonal antibody, an antigen, a lipid, a carbohydrate, a peptide, a protein, a protein complex, an amino acid, a fatty acid, a nucleotide DNA, RNA, PNA, siRNA and microRNA.

In a particularly preferred embodiment of the method, which is subject-matter of the present invention, the one or more biomolecules are proteins.

In a particularly preferred embodiment of the method, which is subject-matter of the present invention, the one or more biomolecules are peptides.

In the method, which is subject-matter of the present invention, the one or more biomolecules present can be analysed in parallel with the aid of an internal standard, wherein the internal standard is comprised of one or more biomolecules from a reference group, which are labelled by one or more molecules according to the invention.

Another subject-matter of the present invention is the use of a molecule and/or a kit according to the invention in a method according to the invention.

Another subject-matter of the present invention is the use of a molecule and/or a kit according to the invention for comparing the expression profiles of biomolecules.

Another subject-matter of the present invention is the use of a molecule and/or a kit according to the invention for performing screenings for diseases related to the aberrant expression of proteins or peptides.

Another subject-matter of the present invention is the use of a molecule and/or a kit according to the invention for performing screenings for cancer.

Another subject-matter of the present invention is the use of a molecule and/or a kit according to the invention for performing screenings for prostate cancer.

Another subject-matter of the present invention is the use of a molecule and/or a kit according to the invention for the discovery of biomarkers.

Another subject-matter of the present invention is the use of a molecule and/or a kit according to the invention for the validation of biomarkers.

Another subject-matter of the present invention is the use of a molecule and/or a kit according to the invention for the quantification of biomarkers.

### Example 1

### Biomarker identification in prostate cancer

Prostate cancer (PCa) is the most common malignancy in European males. In 2002 in Europe, an estimated 225,000 men were newly diagnosed with PCa and about 83,000 died from this disease (http://www-dep.iarc.fr). PCa is diagnosed by histological examination of prostate tissue that is obtained by ultrasound guided transrectal biopsy. Indications for biopsy are predominantly an increased serum prostate specific antigen (PSA) and/or an aberrant digital rectal examination. PSA is the standard diagnostic and prognostic PCa marker. PCa awareness, leading to widespread use of PSA testing has led to a lower tumour stage and grade at the time of diagnosis. However, the use of PSA is associated with certain drawbacks.
1. An increase in PSA can reflect benign as well as malignant prostatic disease, i.e. PSA is not cancer specific, resulting in a negative biopsy rate of 70-80%. This means that a large number of patients is unnecessarily undergoing prostate biopsies.
2. Increased detection leads to the diagnosis of clinically irrelevant tumours (i.e. overdiagnosis) and potentially overtreatment.
3. There is also the problem of patients with clinically localised disease, who have undetected micrometastases leading to symptomatic cancers in due time. Such patients are presently not curable. If they could be identified, they should be considered for adjuvant therapy at the time of primary treatment.
4. Many PCa patients who are not cured by radical therapy will ultimately develop hormone independence and treatment resistance. Early recognition of androgen independence may be an indication for systemic treatment by one of many second line treatments at a phase of low tumour burden. However, at the moment it is impossible to predict therapy resistance at an early stage.

Consequently, there is a growing need for PCa markers that can:
1. Increase the diagnostic specificity.
2. Differentiate between harmless and aggressive tumours.
3. Identify progression towards androgen independence at an early stage.

Serum and urine contain degradation products of extracellular matrix and of benign and malignant cells and their secreted products. These will appear in plasma and urine. Determination of markers in these fluids has certain advantages over the use of tissue markers. Even though the release of PCa-related proteins and/or peptides into plasma and urine incompletely reflects tumour metabolism, these body fluids can easily be obtained. In contrast, prostate tissue sampling requires an invasive procedure (i.e. transrectal ultrasound-guided biopsy). Furthermore, sampling error is an important problem. For example, the tumour can be missed completely causing a false-negative test result. On the other hand, a positive biopsy may not be representative of the tumour, due to the often polyclonal nature of PCa.

### Prostate Specific Antigen (PSA)

Across the world, the suitability of population screening for prostate cancer continues to be highly controversial, fuelled by advocacy in the absence of robust evidence (Frankel S. et al (2003), Lancet, 361(9363), 1122-28). The aim of screening for prostate cancer would be to find potentially life-threatening tumours efficiently and safely among asymptomatic men, at a stage when lesions could be cured by effective treatment, leading to improvements in life expectancy and quality of men's lives. As screening requires intervention in healthy populations, the balance of evidence should convince that the prospect of benefit outweighs harm.

### Example 2

### Identifying potentially treatable tumours

Serum prostate-specific antigen (PSA) testing followed by transrectal ultrasound-guided needle biopsy allows the detection of prostate cancer in some men who might benefit from radical intervention (between 50 and 70 years). Prostate cancer can be found in between 2% (Frankel S. et al (2003), Lancet, 361(9363), 1122-28) and 40% (Thompson, I.M. (2003), The new England Journal of Medicine, 349 (3), 215-24), depending on the intensity of the screening effort - such as the number of PSA tests, level of PSA cutoff value, number and frequency of biopsies undertaken. PSA is not prostate cancer-specific and can be raised in other circumstances, leading to a large number of false-positives (cancer is not found in around 70% of men with raised PSA levels who undergo biopsy). The simple PSA blood test is safe and acceptable, but biopsy can be uncomfortable or painful and carries risks of bleeding and infection. In addition, there will be an unpredictable number of false-negatives who later develop prostate cancer in the presence of a 'normal' PSA test: 36.5% of detectable tumours were identified in men who had PSA levels below 4ng/ml in the European Randomised Screening Trial (ERSPC) (Schröder, F.H. (2000), The Journal of Urology, 163, 806-812).

It is clear that we need better diagnostic tests for prostate cancer that ideally identify and discriminate those cancers, which are destined to progress from those, which are indolent. PSA has been a useful tool to "trigger" biopsy, and is a good way of monitoring men with known cancer, but is not an effective screening test. In the Prostate Cancer Prevention Trial where men had biopsies taken several times, regardless of PSA level, the incidence of prostate cancer was as shown below (Thompson, I. Et al (2004), The new England Journal of Medicine, 350, 2239-2246).
- 6.6% with PSA level 0.5
- 10.1% with PSA 0.6 to 1.0
- 17.0% with PSA 1.1 to 2.0
- 23.9% with PSA 2.1 to 3.0
- 26.9% with PSA 3.1 to 4.0

Even amongst those patients diagnosed with localised prostate cancer, there are further uncertainties. Likelihood and speed of tumour progression are currently impossible to predict. Tumours with high Gleason grades are more likely to progress, but the risk of death within 15 years of diagnosis ranges from 60-80% for those with the highest scores (8-10) to 4-7% with scores of 2-4, and 18-30% for the most common screen-detected score of 6 (Albertsen, P.C. et al (1998), JAMA, 280, 975-80).
Current screening techniques therefore enable tumours to be detected with the potential for cure, but as it is currently impossible to distinguish between indolent and life-threatening lesions there is potential for considerable over-treatment of insignificant disease.

### Example 3

### Parallel screening of 8 patient samples for marker discovery

In order to get relevant information on protein expression differences, which potentially are of clinical relevance, an experimental design on the basis of Figure 4 will be used.

As the use of isotopic tags (e.g., ICAT™) will only allow to discriminate between expression differences of two independent groups on the level of MS, it is necessary to apply an experimental design according to the clinical need that will be addressed.

The experimental design is chosen such, that any expression difference, which appears on the level of MS investigation between the "light" and the "heavy" isotopic labelled groups reflect a potentially relevant discriminating feature between samples from a healthy/control donor or a benign disease versus cancer in local or advanced stage of the disease. The further investigation of this MS signal by MS/MS gives a more detailed answer on the level of differences between the investigated groups.

In case a different clinical question is addressed, the design of the experiment has to be adjusted accordingly. For instance, if the goal is to identify markers for staging of prostate cancer, the design may look like the following:
- group 1: benign donor OR intraepithelial neoplasia (PIN)
- group 2: local PCa disease
- group 3: locally advanced PCa disease
- group 4: aggressive, hormone-refractory PCa disease

After the cleavage/digestion of proteins, many peptides are generated with a free terminal amino group. These groups are reacted with amine-reactive 'purification compounds' containing a 'purification unit' (e.g., biotin, hexa-histidine-tag, others) under appropriate conditions. These 'purification compounds' can be one out of the following (not limited to those):
The isolated N-terminal peptides are further fractionated by standard procedures, like isoelectric focusing, SDS PAGE, two-dimensional gel electrophoresis, size-exclusion chromatography, ion exchange chromatography, reversed-phase HPLC, affinity chromatography, etc.

Each chromatography fraction is further analysed by mass spectrometric techniques (e.g., MALDI, ESI). The nature of peptide peaks in the mass spectrum is identified by fragmentation of the peptide and subsequent analysis of generated fragments by mass spectrometry.

## Claims

1. Molecule for the detection, identification and/or quantification of one or more biomolecules in a sample, wherein said molecule is selected from a group comprising molecules of formula I, II, III, IV and V: 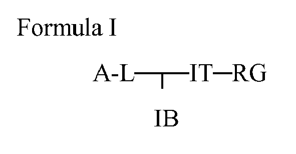 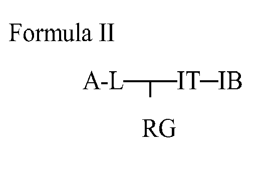 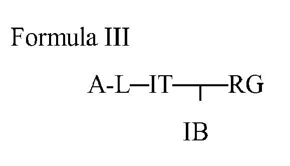
A-L-IB-IT-RG Formula IV 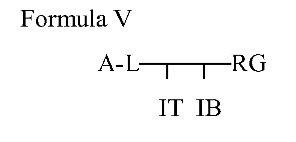
wherein,
• A specifies at least one functional group for the reversible, covalent or non-covalent binding to a support material;
• IB is a group comprising an isobaric tag group;
• IT is a group comprising an isotopic tag group;
• L specifies a linker containing a cleavable moiety; and
• RG is a reactive group for the selective binding of biomolecules, such as, but not limited to proteins and/or peptides.

2. Molecule according to claim 1, wherein A is selected from the group comprising biotin and derivatives thereof, hexa-histidine-tag, amylose binding protein, maltose binding protein, chitin, chitin binding protein, streptavidin, avidin, neutravidin, glutathione-S-transferase and green-fluorescent protein.

3. Molecule according to claims 1 to 2, where L is an aliphatic hydrocarbon chain that contains a cleavable moiety, the latter is selected from the group comprising
• an acid labile moiety,
• a base labile moiety,
• a moiety which is cleavable by UV irradiation,
• a moiety which is cleavable by microwave irradiation,
• a moiety which is cleavable by change of electric potential,
• a moiety which is cleavable by oxidation,
• a moiety which is cleavable by reduction,
• a moiety which can be altered by olefine metathesis and
• a moiety which can be altered by disulfide exchange.

4. Molecule according to claims 1 to 3, where IB comprises an isotopically labelled reporter group and an isotopically labelled counterbalancing group.

5. Molecule according to claims 1 to 4, where IB is labelled with various atoms which occur in more than one stable isotopic form.

6. Molecule according to claims 4 to 5, where IB comprises a predetermined breaking point between the isotopically labelled reporter group and the isotopically labelled counterbalancing group.

7. Molecule according to claims 1 to 6, where IT is an aliphatic hydrocarbon chain.

8. Molecule according to claims 1 to 7, where IT is labelled with various atoms which occur in more than one stable isotopic form.

9. Molecule according to claims 1 to 8, where the group RG, which is reactive towards biomolecules, such as, but not limited to proteins and/or peptides, is selected from the group comprising amino-reactive chemical groups, thiol-reactive chemical groups, hydroxyl-reactive chemical groups and carboxyl-reactive chemical groups.

10. Kit comprising a molecule according to claims 1 to 9.

11. Kit comprising a combinatoric mixture of n · m ≥ 4-8 different molecules according to claims 1 to 9, wherein
• the integer n ≥ 2 specifies the number of differently labelled isotopic tag groups IT, each one having a different molecular weight,
• the integer m ≥ 2-4 specifies the number of differently labelled isobaric tag groups IB, whereby all IB groups have the same molecular weight, while the counterbalancing group of each IB has a different molecular weight, and
• the combinations of the groups IT and IB are chosen such, that the highest possible number of permutations is achieved.

12. Kit comprising parts of a molecule according to claims 1 to 9, whereas one part comprises IB, and another part comprises IT.

13. Method for the detection, identification and/or quantification of one or more biomolecules in a sample, wherein said method comprises the steps of:
a) providing a sample which contains one or more biomolecules;
b) providing a combinatoric mixture of n · in ≥ 4-8 different molecules according to claims 1 to 9, wherein
• the integer n ≥ 2-4 specifies the number of differently labelled isotopic tag groups IT, each one having a different molecular weight,
• the integer m ≥ 4 specifies the number of differently labelled isobaric tag groups IB, whereby all IB groups have the same molecular weight, while the counterbalancing group of each IB has a different molecular weight, and
• the combinations of the groups IT and IB are chosen such, that the highest possible number of permutations is achieved.
c) labelling of the one or more biomolecules from step a) by attachment to the molecules of step b);
d) combining all samples from step c);
e) selecting the one or more biomolecules from the combined sample of step d), under the employment of functional group A of the molecules according to claims 1 to 9, followed by removal of the one or more unbound biomolecules, and thereafter optionally releasing the bound biomolecules from the support material and the elution from the matrix via modification of the linker group by chemical and/or physical methods;
f) detecting, quantifying and/or identifying the one or more labelled biomolecules by means of LC-MS and/or LC-MS/MS.

14. Method according to claim 13 wherein step a) comprises cleaving the one or more biomolecules in said sample.

15. Method according to claims 13 to 14, wherein the one or more biomolecules present are analysed in parallel with the aid of an internal standard, wherein the internal standard is comprised of one or more biomolecules from a reference group, which are labelled by one or more molecules according to claims 1 to 9.

16. Use of a molecule according to claims 1 to 9 or a kit according to claims 10 to 12 for comparing the expression profiles of biomolecules.

17. Use of a molecule according to claims 1 to 9 or a kit according to claims 10 to 12 for performing screenings for diseases related to the aberrant expression of proteins and/or peptides.
